# EUROPÄISCHE PATENTSCHRIFT

(11) **EP 2 735 294 B1**
(45) Veröffentlichungstag und Bekanntmachung des Hinweises auf die Patenterteilung: **09.03.2016**
(21) Anmeldenummer: 13192705.5
(22) Anmeldetag: 13.11.2013
(51) Int. Cl.: A61G 12/00, A61B 50/13

(54) **Medizinisches Gerät sowie medizinisches Gerätesystem**
Medical device and medical device system
Appareil médical et système d'appareil médical

(30) Priorität: 22.11.2012 DE 102012111260
(43) Veröffentlichungstag der Anmeldung: 28.05.2014
(73) Patentinhaber: Karl Storz GmbH & Co. KG, 78532 Tuttlingen (DE)
(72) Erfinder: Emmerich, Bernd, 78532 Tuttlingen (DE); Jansche, Andreas, 78532 Tuttlingen (DE)

(56) Entgegenhaltungen:
- WO-A1-2013/102494
- DE-A1- 2 348 686
- DE-A1-102009 024 335
- US-A1- 2003 037 375
- US-A1- 2012 203 377
- US-B1- 7 708 586

## Beschreibung

Die Erfindung betrifft ein medizinisches Gerät zur Verwendung in einer medizinischen Anwendungsumgebung, das Haltemittel zum Halten des Geräts an einer der Anwendungsumgebung zugeordneten Trägervorrichtung sowie Anschlussmittel zum Anschließen des Geräts an mindestens eine elektrische Verbindungsleitung umfasst. Die Erfindung betrifft ferner ein medizinisches Gerätesystem, das eine Mehrzahl derartiger medizinischer Geräte sowie eine Trägervorrichtung für die Mehrzahl der medizinischen Geräte umfasst.

Im medizinischen Bereich, insbesondere bei chirurgischen Operationen oder auch in der Intensivmedizin, werden häufig vielfältige elektrische und elektronische medizinische Geräte benötigt, um beispielsweise Endoskope oder chirurgische Instrumente mit Energie zu versorgen und zu steuern oder auch um Vitalfunktionen eines Patienten zu überwachen. Derartige Geräte sollen möglichst platzsparend in der Nähe des Patienten angeordnet werden können, ohne den Zugang zum Patienten zu behindern und sollen andererseits einfach und sicher montierbar, anschließbar, bedienbar und ggf. rasch austauschbar sein.

Es ist bekannt, derartige medizinische Geräte beispielsweise auf einem Fahrwagen, auf einem stationären OP-Möbel oder auch auf einer oder mehreren an einem an der Decke eines Operationssaals befestigten schwenkbaren Deckenarm gehaltenen Konsolen aufzustellen. Hierbei sind die Geräte jedoch nicht ausreichend gegen Herunterfallen sowie gegen unbefugtes Entnehmen gesichert. Die zum Aufstellen der Geräte notwendigen Konsolen haben selbst einen erheblichen Raumbedarf und erhöhen das Gesamtgewicht der Anordnung. Die elektrischen Anschlüsse für Netzspannung und Datenleitungen sind frei zugänglich angeordnet, wobei die entsprechenden Leitungen einzeln an die jeweils entsprechenden Anschlüsse angeschlossen werden müssen. Zum Ein- und Ausstecken elektrischer Leitungen oder von Lichtleitkabeln sowie bei weiteren Bedienhandlungen sind häufig zwei Hände notwendig, um die notwendige Gegenkraft aufbringen zu können und ein ungewolltes Verschieben des Geräts zu vermeiden. Die frei zugänglichen Leitungen und die große Gesamtoberfläche der Gerätegehäuse verursachen einen erhöhten Reinigungsaufwand.

Aus EP 2 058 911 A1 ist eine medizinische Versorgungseinheit zur Stromversorgung und Datenübertragung bei medizinischen Apparaten bekannt, die einen langgestreckten Träger mit einer sich entlang des Trägers erstreckenden und mindestens zwei durchgehende, elektrisch isolierte Leiterschienen aufweisende Kopplungseinrichtung umfasst. Die Kopplungseinrichtung ist als Hohlschiene ausgebildet, auf deren Innenseite die Leiterschienen angeordnet sind. Ein Modulbauteil weist ein Kopplungsstück auf, das den Kontakt mit den Leiterschienen herstellt. Ein Austausch eines einzelnen medizinischen Gerätes ist hierbei nicht ohne weiteres möglich. Ferner sind das von der Kopplungseinrichtung aufnehmbare Gewicht sowie die Zahl und Art der auf diese Weise herstellbaren Verbindungen zur Stromversorgung und Datenübertragung relativ eng begrenzt.

Gemäß DE 699 35 622 T2 umfasst eine Anschlussplatte zum Aufnehmen eines Instruments eine Platte, eine in einem Abstand von der Platte auf der Platte montierte Schiene und einen in der Platte gesicherten Signalport, der mit einem elektrischen Port des Gehäuses des Instruments gekoppelt werden kann. Auch hierbei ist das Gewicht der aufnehmbaren Instrumente eng begrenzt. Ferner sind die Instrumente an der Anschlussplatte nicht ausreichend gegen Herunterfallen oder unbefugte Entnahme gesichert.

In der US2012/0203377 ist ein Wagen zur Aufnahme mehrerer Arzneimittelbehälter offenbart. Diese werden an einer darüber angeordneten Arbeitsfläche und miteinander über Vorsprünge verbunden. Sie sind außerdem mit einer Säule des Wagens über Anschlüsse elektrisch verbunden. Ein Stift an jeder Kassette ermöglicht eine korrekte Ausrichtung der Kassetten entlang der Säule.

Die mechanische und die elektrische Verbindung muss hier jeweils separat hergestellt werden.

Die US7,708,586B1 beschreibt einen Gewindebolzen zur Befestigung einer beleuchteten Steckdose an einem Paneel.

US2003/0037375A1 offenbart eine Patientenliege mit mehreren Aufnahmen für senkrechte Stangen, an denen Monitore über der Liege aufgehängt werden können. Die Stangen stellen eine elektrische Verbindung mit den Monitoren her.

Es ist Aufgabe der vorliegenden Erfindung, ein gattungsgemäßes medizinisches Gerät sowie ein medizinisches Gerätesystem anzugeben, das die oben genannten Nachteile nicht aufweist.

Diese Aufgabe wird durch ein medizinisches Gerät gemäß Anspruch 1 sowie durch ein medizinisches Gerätesystem gemäß Anspruch 9 gelöst.

Vorteilhafte Weiterbildungen der Erfindung ergeben sich aus den Unteransprüchen.

Ein erfindungsgemäßes medizinisches Gerät ist für den Einsatz in einer medizinischen Anwendungsumgebung ausgebildet, insbesondere in einem Operationssaal einer Klinik oder Tagesklinik, aber auch beispielsweise in einem Vorbereitungs- oder Untersuchungsraum einer ärztlichen Einrichtung. Ein derartiges Gerät ist insbesondere ein Versorgungs- oder Steuerungsgerät für ein chirurgisches Instrument oder ein Endoskop, etwa ein HF-Generator, eine Lichtquelle oder eine Spül- oder Insufflationspumpe. Das Gerät kann aber auch beispielsweise ein Monitor für Vitalparameter des Patienten oder ein Bedienteil einer zentralen OP-Steuerung sein.

Das medizinische Gerät weist Haltemittel zum mechanischen Halten des Geräts an einer der Anwendungsumgebung zugeordneten senkrechten Trägervorrichtung auf. Die Trägervorrichtung kann ein fahrbarer Gerätewagen, ein an einer Raumdecke befestigter Deckenversorgungsarm oder ein stationäres OP-Möbel sein oder kann an einem fahrbaren Wagen, an einem Deckenarm, an einem OP-Möbel oder auch an einer Wand eines Operationssaales angeordnet bzw. darin integriert sein. Die Trägervorrichtung kann insbesondere eine platten- oder schienenförmig ausgebildete Befestigungseinrichtung umfassen, die mit den Haltemitteln des Geräts zum Halten des Geräts zusammenwirkt.

Das medizinische Gerät weist ferner Anschlussmittel zum Anschließen des Geräts an mindestens eine elektrische Verbindungsleitung auf. Die elektrische Verbindungsleitung kann der Stromversorgung des Geräts und/oder der Datenübertragung dienen und kann ein- oder mehradrig ausgebildet sein. Die elektrische Verbindungsleitung kann Teil der Trägervorrichtung sein oder unabhängig von dieser geführt sein. Vorzugsweise ist die elektrische Verbindungsleitung der Anwendungsumgebung zugeordnet und dient etwa zum Verbinden des Geräts mit einer Spannungsversorgung und mit einer zentralen OP-Steuerung.

Erfindungsgemäß sind die Haltemittel als mindestens ein mit einer Tragstruktur des Geräts verbundener Tragbolzen ausgebildet. Die Tragstruktur des Geräts kann beispielsweise ein Rahmen des Geräts oder auch ein selbsttragendes Gehäuse des Geräts sein. Der Tragbolzen steht von der Tragstruktur bzw. vom Gerät ab, um die Montage des Geräts an der Trägervorrichtung zu vereinfachen. Das Gerät weist ein im Wesentlichen geschlossenes Gehäuse auf; in diesem Fall steht der Tragbolzen von einer Wand des Gehäuses, insbesondere einer Rück- oder Seitenwand des Gehäuses, nach außen ab.

Erfindungsgemäß weist der Tragbolzen die Anschlussmittel zum Anschließen des Geräts an die mindestens eine elektrische Verbindungsleitung auf. Zusätzlich können weitere Anschlussmittel, beispielsweise zum Anschluss einer Glasfaserleitung oder einer Gas- oder Flüssigkeitsleitung, vorhanden sein, die entsprechende optische Bauteile bzw. Dichtelemente umfassen können und die vorzugsweise ebenfalls von dem mindestens einen Tragbolzen umfasst werden. Zur Verbindung mit einem von den medizinischen Gerät versorgten oder gesteuerten Funktionselement, etwa einem Sensor, einem chirurgischen Instrument oder einem Endoskop, können außerdem weitere Anschlußmittel vorgesehen sein, die nicht vom Tragbolzen umfasst werden und die vorzugsweise an einer Frontseite des Geräts bzw. des Gehäuses angeordnet und für einen Bediener dadurch besonders leicht zugänglich sind.

Erfindungsgemäß wird somit ein medizinisches Gerät geschaffen, bei dem sowohl das mechanische Halten des Geräts als auch das Herstellen einer elektrischen Verbindung in einer medizinischen Anwendungsumgebung vereinfacht ist. Hierdurch kann das medizinische Gerät rasch montiert und gegen Herunterfallen gesichert und mit den zum Betrieb notwendigen elektrischen Verbindungen versehen werden. Dadurch, dass die Haltemittel als min destens ein mit der Tragstruktur des Geräts verbundener Tragbolzen ausgebildet sind, wird ein mechanisches Halten des Geräts alleine über den mindestens einen Tragbolzen ermöglicht und hierdurch die Montage des Geräts an der Trägervorrichtung vereinfacht. Dadurch, dass das Gerät fest an der Trägervorrichtung gehalten wird, müssen beim Ein- und Ausstecken von Steckern nicht mehr zwangsläufig beide Hände zu Hilfe genommen werden. Ferner wird dadurch, dass der Tragbolzen die Anschlussmittel zum Anschließen des Geräts an die mindestens eine elektrische Verbindungsleitung aufweist, das Anschließen des Geräts an die Verbindungsleitung vereinfacht. Sofern das medizinische Gerät ein Gehäuse aufweist, wird hierdurch außerdem die Zahl der notwendigen Unterbrechungen des Gehäuses verringert, was aus hygienischen Gründen in einer medizinischen Anwendungsumgebung vorteilhaft ist.

Vorzugsweise weist das medizinische Gerät nur einen einzigen derartigen Tragbolzen auf, der zum Halten des Geräts an der Trägervorrichtung ausgebildet ist und der alle notwendigen Anschlussmittel zum Anschließen des Geräts an die mindestens eine elektrische Verbindungsleitung umfasst. Die mechanische und die elektrische Verbindung können somit gemeinsam über einen entsprechend stabil ausgeführten Tragbolzen als einziges mechanisch-elektrisches Kopplungsmittel hergestellt werden. Hierdurch wird sowohl die mechanische Montage als auch das Herstellen der zum Betrieb des Geräts notwendigen elektrischen Verbindungen weiter vereinfacht. Ferner wird dadurch, dass die Zahl der Handhabungsschritte zur mechanischen Montage und zum elektrischen Verbinden und der hierfür zu handhabenden Bereiche des Geräts verringert ist, die Zahl der Fehlermöglichkeiten verringert; da mit jedem Handhabungsschritt auch eine Kontamination des Geräts verbunden sein kann, wird hierdurch auch das hygienische Risiko verringert bzw. die Reinigung erleichtert. Insbesondere bei Geräten mit einem hohen Eigengewicht können andererseits auch mehrere Tragbolzen vorgesehen sein.

Gemäß einer bevorzugten Ausführungsform der Erfindung sind die Anschlussmittel als an einer vom Gerät entfernten Stirnseite des Tragbolzens angeordnete Steckkontakte ausgebildet. Die Steckkontakte können beispielsweise Kontaktstifte sein, die in eine oder mehrere Buchsen der Verbindungsleitung eingreifen können. Die Steckkontakte können aber auch beispielsweise Buchsen bilden, die für das Aufnehmen von der Verbindungsleitung zugeordneten Kontaktstiften vorgesehen sind. Der Tragbolzen kann auch sowohl Kontaktstifte als auch buchsenartige Steckkontakte aufweisen, die zum Zusammenwirken mit komplementären Buchsen bzw. Kontaktstiften der Verbindungsleitung ausgebildet sind. In vorteilhafter Weise sind die Steckkontakte des Tragbolzens in einer Längsrichtung des Tragbolzens gerichtet, so dass ein Aufschieben entsprechender Steckkontakte der Verbindungsleitung in Längsrichtung des Tragbolzens erfolgen kann. Besonders bevorzugt ist es, dass die Steckkontakte des Tragbolzens zum Zusammenwirken mit einem einzigen Stecker der Verbindungsleitung, der entsprechende Kontaktstifte und/oder Buchsen aufweist und der in der Längsrichtung des Tragbolzens auf diesen aufschiebbar ist, angeordnet sind. Hierdurch wird das Anschließen des Geräts an die elektrische Verbindungsleitung weiter vereinfacht.

Weiterhin ist es bevorzugt, dass der Tragbolzen zumindest abschnittsweise ein Außengewinde aufweist. Durch das Außengewinde wird beispielsweise das Aufschrauben einer Mutter zum Sichern an der Trägervorrichtung ermöglicht. Ferner kann das Außengewinde zur Befestigung des Tragbolzens an der Tragstruktur des Geräts dienen. Hierdurch wird auf einfache Weise eine sichere und feste Fixierung des Tragbolzens an dem Gerät und an der Trägervorrichtung ermöglicht.

Gemäß einer weiteren, nicht beanspruchten Ausführungsform der Erfindung weist die Tragstruktur des Geräts ein Langloch auf. Das Langloch kann beispielsweise als Schlitz eines Gehäuses, insbesondere als Schlitz eines selbsttragenden Gehäuses, ausgebildet sein; der Schlitz ist insbesondere bei einer üblichen Aufstellung des Geräts ein horizontaler Längsschlitz des Gehäuses. Der Tragbolzen ist in dem Langloch verschiebbar und in einer Mehrzahl von Positionen, vorzugsweise in beliebigen Positionen, an der Tragstruktur fixierbar. Hierdurch wird es ermöglicht, das Gerät in einer gewünschten Position relativ zur Trägervorrichtung zu positionieren, insbesondere in horizontaler Richtung, und in dieser Position zu fixieren.

Im gerätenahen Bereich des Tragbolzens weist dieser in vorteilhafter Weise mindestens zwei, vorzugsweise genau zwei, paarweise einander gegenüberliegende erste Abflachun gen auf, die mit dem Langloch derart zusammenwirken, dass der Tragbolzen innerhalb des Langlochs verschiebbar ist und verdrehgesichert geführt wird. Die ersten Abflachungen erstrecken sich zumindest über einen Teilbereich der Länge des Tragbolzens parallel zu seiner Längsachse. In den nicht abgeflachten Bereichen kann der Tragbolzen ein Außengewinde aufweisen, mit dem der Tragbolzen beispielsweise mit Hilfe einer Mutter und einer Gegenmutter an einer Mehrzahl von Positionen im Langloch verschraubbar sein kann. Vorzugsweise ist der Tragbolzen stufenlos verstellbar. Hierdurch wird auf einfache Weise eine an konkrete Raumerfordernisse anpassbare Befestigungsmöglichkeit des Geräts an der Trägervorrichtung geschaffen.

Weiterhin ist es bevorzugt, dass der Tragbolzen in seinem gerätefernen Bereich mindestens zwei einander jeweils gegenüberliegende zweite Abflachungen aufweist, die sich in Längsrichtung des Tragbolzens bis zu seinem vom Gerät abgewandten Ende erstrecken können. Vorzugsweise weist der Tragbolzen genau zwei einander gegenüberliegende zweite Abflachungen auf. Hierdurch wird in ähnlicher Weise wie oben beschrieben eine Verschiebbarkeit in einem entsprechenden Langloch bzw. Schlitz der Trägervorrichtung ermöglicht. Die nicht abgeflachten Bereiche können ein Außengewinde aufweisen, so dass der Tragbolzen beispielsweise mit einer Mutter und einer Gegenmutter an einer mit einem derartigen Langloch versehenen Befestigungsplatte der Trägervorrichtung befestigt werden kann. Hierdurch wird eine Fixierung in einer Mehrzahl von Positionen ermöglicht, vorzugsweise an einer beliebigen Position innerhalb des Langlochs, wodurch das Gerät auf besonders einfache Weise in unterschiedlichen Positionen an der Trägervorrichtung montiert und fixiert werden kann.

In besonders bevorzugter Weise sind zwei erste und zwei zweite Abflachungen vorgesehen, wobei die ersten und die zweiten Abflachungen um ca. 90° bezüglich einer Längsachse des Tragbolzens zueinander versetzt sind. Hierdurch wird es ermöglicht, das Gerät, dessen Tragstruktur ein sich in einer ersten Richtung erstreckendes Langloch aufweist, an einer Trägervorrichtung, die ein sich in einer zweiten, zur ersten im Wesentlichen senkrechten Richtung erstreckendes Langloch aufweist, in einer Vielzahl möglicher Positionen zu befestigen. Insbesondere kann die erste Richtung horizontal und die zweite Richtung vertikal gerichtet sein, so dass das Gerät an einer Befestigungsplatte mit einem vertikalen Langloch in beliebiger Höhe und durch Verschiebung entlang dem Langloch des Geräts in einer gewünschten horizontalen Position fixiert werden kann.

Ein erfindungsgemäßes Gerätesystem umfasst mindestens zwei medizinische Geräte, die wie oben beschrieben ausgebildet sind, sowie eine Trägervorrichtung für die Geräte. Die medizinischen Geräte können insbesondere Teil eines modularen Systems sein und durch ihre Zusammenstellung und Konfiguration an unterschiedliche Anforderungen, etwa an unterschiedliche Operationsgebiete, anpassbar sein. Die Trägervorrichtung weist eine mit mindestens einem Durchbruch versehene Befestigungsplatte auf, wobei die Tragbolzen der medizinischen Geräte zum Einsetzen in den Durchbruch und zum Befestigen jeweils an einem dem Durchbruch benachbarten Bereich der Befestigungsplatte ausgebildet sind, so dass die medizinischen Geräte an der Befestigungsplatte gehalten werden können. Die Befestigungsplatte kann auch beispielsweise als Befestigungsschiene ausgestaltet sein. Insbesondere ist der Tragbolzen jeweils derart ausgebildet, dass er mit seinem vom Gerät entfernten Endbereich durch den Durchbruch hindurchgeführt werden und mit einem hinter den Durchbruch greifenden Befestigungsmittel an der Befestigungsplatte der Trägervorrichtung fixiert werden kann. Alternativ kann es der Tragbolzen derart ausgebildet sein, dass er mit seinem vom Gerät entfernten Endbereich in den Durchbruch hineingeführt werden kann und ohne ein hinter den Durchbruch greifendes Befestigungsmittel kraft- oder formschlüssig in dem Durchbruch gehalten ist; in diesem Fall kann anstelle des Durchbruchs ein Sackloch vorgesehen sein, und die Trägervorrichtung kann etwa ein Teil einer Wand eines Raums sein.

Die Trägervorrichtung kann beispielsweise ein verschiebbarer Wagen, ein OP-Möbel oder ein an einer Decke eines Operationssaals angebrachter Deckenarm sein oder an einem solchen Wagen, an einem OP-Möbel oder einem Deckenarm oder auch an einer Wand der medizinischen Anwendungsumgebung befestigbar sein oder mit dieser integriert ausgebildet sein. Die Trägervorrichtung kann die mindestens eine elektrische Verbindungsleitung eines jeweiligen Geräts umfassen, oder die Verbindungsleitung kann separat von der Trägervorrichtung geführt sein. Vorzugsweise weist die jeweilige elektrische Verbindungsleitung einen Stecker auf, der mit den dem Anschlussbolzen zugeordneten Anschlussmitteln zum Herstellen aller zum Betrieb des Geräts notwendigen elektrischen Verbindungen zusammenwirkt. Die Verbindungsleitung kann der medizinischen Anwendungsumgebung zugeordnet sein und beispielsweise zur Spannungsversorgung des betreffenden Geräts und dem Datenaustausch mit einer zentralen OP-Steuerung dienen. Die Verbindungsleitung kann auch der Trägervorrichtung zugeordnet sein und beispielsweise mit einer Versorgungs- und Datenleitung der Anwendungsumgebung verbindbar sein. Die Verbindungsleitung kann auch zur Verbindung eines Geräts mit einem oder mehreren weiteren Geräten dienen.

Das erfindungsgemäße Gerätesystem ermöglicht auf einfache und sichere Weise eine Befestigung der medizinischen Geräte in einer an den jeweiligen Bedarf angepassten Weise sowie eine gleichzeitige Schaffung der notwendigen elektrischen Verbindungen.

Vorzugsweise ist der Durchbruch der Befestigungsplatte der Trägervorrichtung ein Loch eines Lochrasters oder ein Langloch. Durch die Anordnung der Durchbrüche in Form eines Lochrasters kann eine feste Anordnung von aufeinander horizontal oder vertikal gestapelten Geräten, die standardisierte Abmessungen aufweisen, und somit eine besonders einfache und exakte Montage an der Trägervorrichtung gewährleistet werden. Ein Langloch ermöglicht eine stufenlose Anpassung der Befestigungsposition des Geräts an unterschiedliche Anforderungen, insbesondere an unterschiedliche Höhen der Geräte, mit denen es einen Stapel bilden kann. Insbesondere kann ein Langloch in der Befestigungsplatte in vertikaler Richtung gerichtet sein, um eine Anordnung von Geräten in unterschiedlichen Höhen und etwa eine Stapelung von Geräten unterschiedlicher Höhen in vertikaler Richtung zu ermöglichen.

Vorzugsweise weist das medizinische Gerätesystem eine Mutter auf, die auf einen durch den Durchbruch hindurchführbaren Bereich des Tragbolzens, insbesondere auf ein Außengewinde des Tragbolzens, von dem vom Gerät entfernten Ende her aufschraubbar ist. Zur weiteren Verbesserung der Fixierung kann die Mutter mit einem Flansch versehen sein und/oder es kann eine Unterlegscheibe vorgesehen sein, wobei der Flansch bzw. die Unterlegscheibe hinter den Durchbruch greift. In besonders bevorzugter Weise ist die Mutter gleichzeitig zur Sicherung eines Steckers der Verbindungsleitung ausgebildet, der auf an der vom Gerät entfernten Stirnseite des Tragbolzens angeordnete Steckkontakte aufsetzbar ist. Hierdurch kann in einem einzigen Arbeitsschritt sowohl eine sichere mechanische Fixierung an der Trägervorrichtung als auch eine gesicherte elektrische Verbindung mit der mindestens einen Verbindungsleitung erreicht werden. Die Mutter und die Unterlegschiebe können Teil des medizinischen Gerätesystems sein.

Gemäß einer bevorzugten Ausführungsform der Erfindung sind die medizinischen Geräte des medizinischen Gerätesystems stapelbar ausgebildet, wobei eine Ober- und eine Unterseite des Geräts derart geformt sind, dass ein erstes Gerät mit seiner Unterseite formschlüssig auf die Oberseite eines zweiten Geräts aufsetzbar ist. Das Gerät kann dabei beispielsweise horizontal oder vertikal stapelbar sein, wobei sich die Begriffe "Oberseite" und "Unterseite" auf die Stapelrichtung beziehen. So können derartige Geräte etwa auf einer waagrechten Konsole oder auf einem Fahrwagen in senkrechter Richtung stapelbar sein, während solche Geräte an einer Deckenversorgungseinrichtung hängend in senkrechter oder waagrechter Richtung stapelbar sein können. Hierdurch wird eine sichere und platzsparende Anordnung der Geräte ermöglicht.

Weiterhin ist es bevorzugt, dass das Gerät ein im Wesentlichen allseitig geschlossenes Gehäuse aufweist und an einer Unterkante einer Vorderseite des Gehäuses eine vorstehende Lippe trägt. Die vorstehende Lippe eines ersten Geräts überlappt die Oberkante einer Vorderseite des Gehäuses eines bei einem vertikalen Stapel darunter angeordneten zweiten Geräts, so dass ein Eindringen von Flüssigkeiten zwischen die Geräte vermieden wird. Derartige Flüssigkeiten können beispielsweise Blut oder Spülflüssigkeit sein, die sich aufgrund von Undichtigkeiten oder beim Anbringen oder Entfernen entsprechender Schläuche einer Pumpe an der Vorderseite eines als Pumpe ausgebildeten Geräts niederschlagen können. Dadurch, dass sich die Geräte zumindest an ihrer Vorderseite dachziegelartig überlappen, wird die Reinigung der Geräte vereinfacht.

Gemäß einer bevorzugten Ausführungsform der Erfindung weist das Gehäuse des ersten und des zweiten Geräts jeweils eine Rückwand und/oder eine Seitenwand auf, wobei aus der Rück- bzw. Seitenwand der Tragbolzen des jeweiligen Geräts hervorsteht. Die Geräte können somit an einer Befestigungsplatte, etwa einer Befestigungsschiene, montiert und fixiert werden, die rückseitig bzw. seitlich der Geräte angeordnet ist. Hierdurch wird eine sichere mechanische Halterung und eine elektrische Verbindung ermöglicht, die die Bedienbarkeit von Bedienelementen, die auf einer Vorderseite des jeweiligen Gehäuses angeordnet sind, nicht beeinträchtigt. Insbesondere durch eine seitliche Anordnung der Tragbolzen und der Befestigungsplatte wird darüber hinaus eine besonders gute Zugänglichkeit der Tragbolzen ermöglicht, wodurch die mechanische Fixierung und die Herstellung der elektrischen Verbindung weiter vereinfacht werden.

Eine Haltevorrichtung für ein medizinisches Gerät umfasst mindestens einen Tragbolzen zum Halten eines medizinischen Geräts und eine Trägervorrichtung, die wie oben beschrieben ausgebildet sind, sowie ggf. eine Mutter und eine Unterlegscheibe zum Fixieren des Tragbolzens an der Befestigungsplatte der Trägervorrichtung. Die Haltevorrichtung kann ferner Mittel zum Befestigen des Tragbolzens an dem medizinischen Gerät umfassen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen in schematischer Form:
Fig. 1 eine als Fahrwagen ausgebildete Trägervorrichtung mit einer Befestigungsplatte und zwei medizinischen Geräten gemäß einem ersten Ausführungsbeispiel der Erfindung;
Fig. 2 eine Schnittdarstellung eines medizinischen Geräts und einer Befestigungsplatte gemäß dem ersten Ausführungsbeispiel;
Fig. 3 eine an einem Deckenarm befestigbare Trägervorrichtung mit einer Befestigungsplatte gemäß dem ersten Ausführungsbeispiel;
Fig. 4 eine als Fahrwagen ausgebildete Trägervorrichtung mit einer Befestigungsplatte und drei medizinischen Geräten gemäß einem zweiten Ausführungsbeispiel der Erfindung;
Fig. 5 eine an einem Deckenarm befestigte Trägervorrichtung mit einer Befestigungsplatte gemäß dem zweiten Ausführungsbeispiel;
Fig. 6 eine Detailansicht eines medizinischen Geräts und einer Befestigungsplatte gemäß dem zweiten Ausführungsbeispiel;
Fig. 7 eine Teilansicht einer Befestigungsplatte und zweier medizinischer Geräte gemäß dem zweiten Ausführungsbeispiel;
Fig. 8 eine Teilansicht eines medizinischen Geräts, einer Befestigungsplatte und eines Steckers einer Verbindungsleitung gemäß dem zweiten Ausführungsbeispiel;
Fig. 9 eine Mutter zum Aufschrauben auf einen Tragbolzen gemäß einem Ausführungsbeispiel der Erfindung;
Fig. 10 ein medizinisches Gerätesystem gemäß dem zweiten Ausführungsbeispiel.

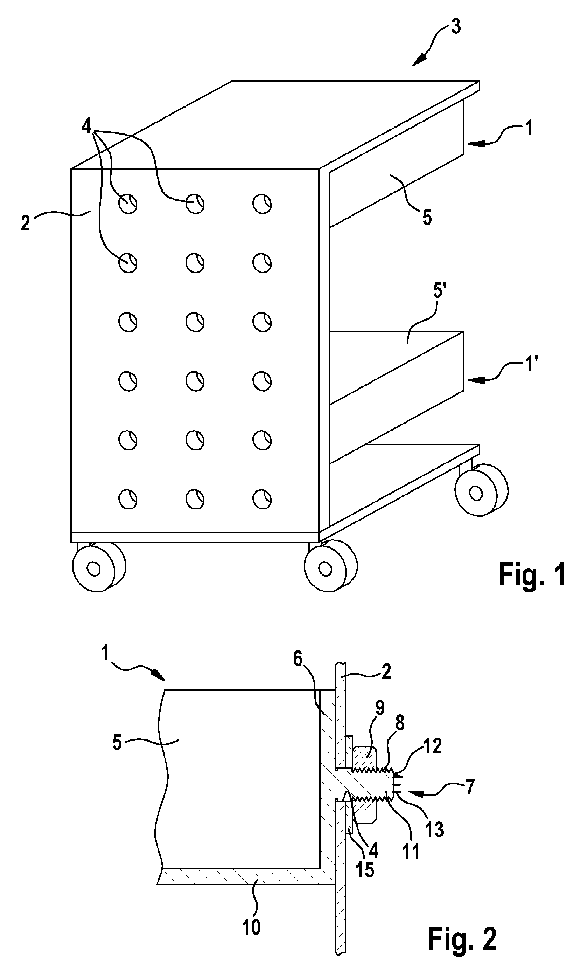

In Fig. 1 ist in vereinfachter Form beispielhaft eine Trägervorrichtung mit daran gehaltenen Geräten 1,1' dargestellt. Die Trägervorrichtung umfasst eine senkrecht stehende Befestigungsplatte 2 und ist als mit Rollen fahrbarer Wagen 3 ausgebildet. Die medizinischen Geräte 1,1' sind beispielsweise Versorgungs- und Steuerungsgeräte für chirurgische Instrumente und/oder Endoskope. Die Befestigungsplatte 2 weist ein Lochraster mit kreisförmigen durchgehenden Löchern 4 auf. An der Befestigungsplatte 2 sind in dem in Fig. 1 beispielhaft gezeigten Zustand zwei Geräte 1,1' befestigt. Hierfür ist jeweils mindestens ein Tragbolzen, der von einer Rückwand des Gehäuses 5,5' des Geräts 1,1' absteht, durch mindestens eines der Löcher 4 eingesetzt und an der Befestigungsplatte 2 verschraubt (in Fig. 1 nicht gezeigt). Die Abmessungen der Geräte 1,1', insbesondere die Höhe, und das Rastermaß der Abstände zwischen den Löchern 4 sind aneinander angepasst. Wie in der Darstellung der Fig. 1 deutlich wird, ist auf einfache Weise ein separater Austausch der Geräte 1,1' sowie weiterer Geräte, die an den frei gebliebenen Positionen unter bzw. zwischen den Geräten 1,1' befestigt und angeschlossen werden können, für Konfigurations- und Servicearbeiten möglich. Zusätzlich zu den Gehäusen 5, 5' kann ein Um-Gehäuse vorgesehen sein, um die Geräte 1,1' zusätzlich gegen Kontamination zu schützen (nicht dargestellt).

Wie in Fig. 2 schematisch in einer Schnittdarstellung gezeigt, steht aus einer Rückwand 6 des Gehäuses 5 des Geräts 1 ein Tragbolzen 7 hervor, der in das Loch 4 der Befestigungsplatte 2 eingesetzt ist. Der Tragbolzen 7 weist ein Außengewinde 8 auf, auf das eine Mutter 9 aufgeschraubt werden kann. Durch Festziehen der Mutter 9 gegen die Befestigungsplatte 2 kann der Tragbolzen 7 und damit das Gerät 1 fest an der Befestigungsplatte 2 fixiert werden, wobei zur Verbesserung der Krafteinleitung in die Befestigungsplatte 2 zwischen der Mutter 9 und der Befestigungsplatte eine Unterlegscheibe 15 angeordnet sein kann. Die Rückwand 6 des Gehäuses 5 ist für ein sicheres Halten des Geräts 1 verstärkt ausgebildet und bildet, wie in Fig. 1 angedeutet, mit einer Unterseite 10 des Gehäuses 5 eine Tragstruktur des Geräts 1. Die Rückwand 6 und die Unterseite 10 des Gehäuses 5 sind entsprechend versteift, etwa als selbsttragendes Chassis, um das Gewicht des Geräts 1 sowie die bei der Bedienung auftretenden Kräfte aufnehmen zu können. Der Tragbolzen 7 ist, wie auch die Befestigungsplatte 2, ebenfalls zum Aufnehmen der notwendigen Kräfte ausgebildet und kann beispielsweise einen rohrförmigen Bolzenkörper 11 mit entsprechender Wandstärke aufweisen. Der Bolzenkörper 11 ist mit der Rückwand 6 des Gehäuses 5 einstückig ausgebildet oder mit dieser verschweißt.

Wie in Fig. 2 ebenfalls zu erkennen ist, trägt der Tragbolzen 7 an seiner vom Gerät 1 abgewandten Stirnseite 12 eine Mehrzahl von elektrischen Kontakten, die beispielsweise als Kontaktstifte 13 ausgebildet sind, die in axialer Richtung des Tragbolzens 7 gerichtet sind. Auf die Kontaktstifte 13 ist ein Stecker einer in Fig. 2 nicht dargestellten elektrischen Verbindungsleitung mit entsprechenden Buchsen aufsteckbar. Elektrische Leitungen, die mit den Kontaktstiften 13 in Verbindung stehen, können innerhalb des Tragbolzens 7 zu entsprechenden Kontakten innerhalb des Geräts 1 geführt sein, oder es können auf der dem Gerät zugewandten Stirnseite des Tragbolzens 7 weitere Kontakte zur Verbindung mit entsprechenden geräteinternen elektrischen Leitungen angeordnet sein (in Fig. 2 nicht gezeigt).

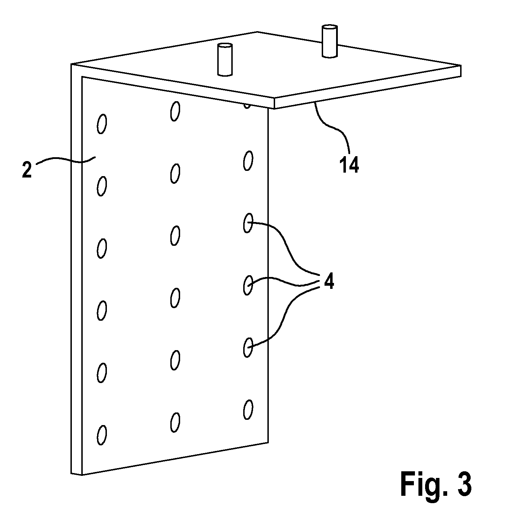

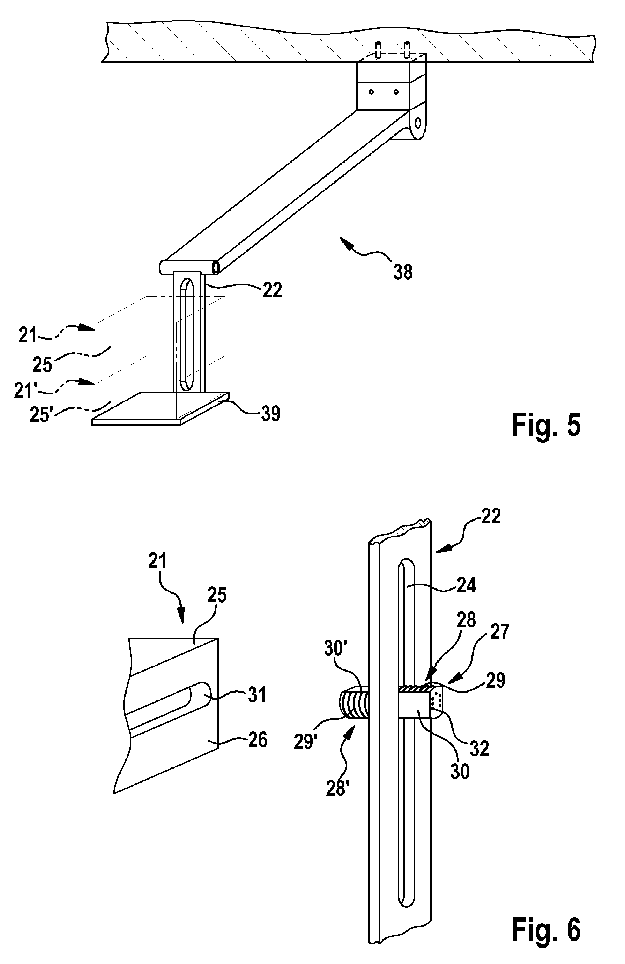

In Fig. 3 ist beispielhaft die Anbringung einer wie in Fig. 1 ausgebildeten Befestigungsplatte 2 an einem Deckenhalter 14, der etwa an einem an einer Raumdecke angeordneten schwenkbaren Deckenversorgungsarm (s. Fig. 5) oder auch direkt an einer Raumdecke angebracht werden kann, dargestellt.

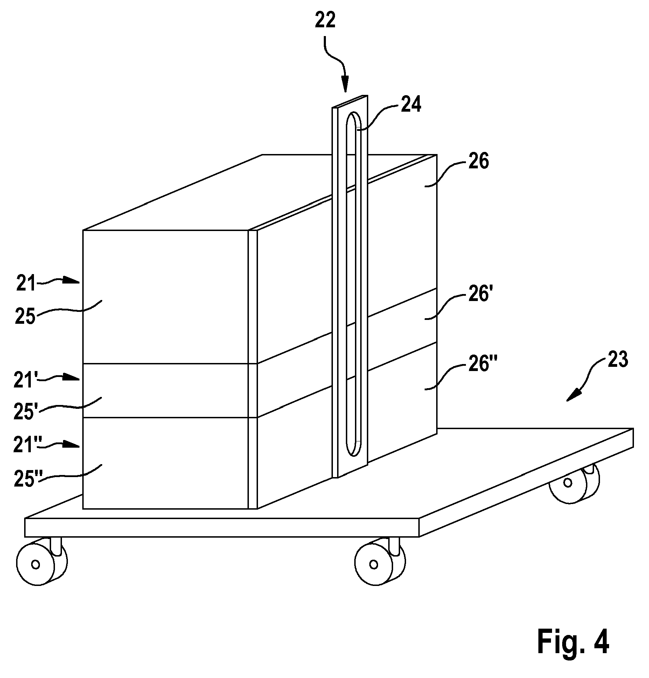

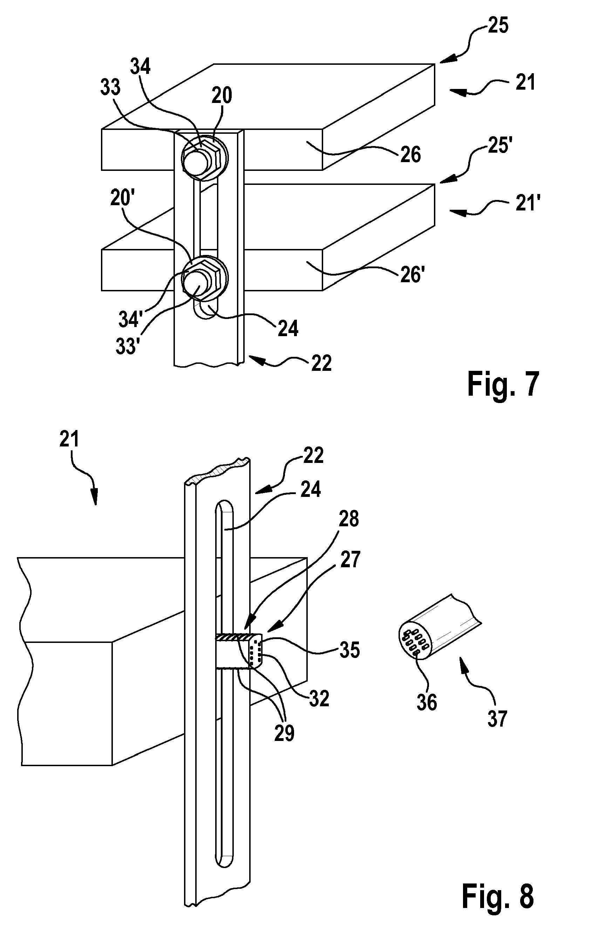

Gemäß dem in Fig. 4 dargestellten weiteren Ausführungsbeispiel umfasst ein erfindungsgemäßes medizinisches Gerätesystem eine Mehrzahl von medizinischen Geräten 21, 21' 21" sowie eine Trägervorrichtung, die eine als Befestigungsschiene ausgebildete Befestigungsplatte 22 umfasst und die als mit Rollen versehener fahrbarer Wagen 23 ausgebildet ist. Die Befestigungsplatte 22 ist senkrecht stehend auf einer Grundplatte des Wagens 23 befestigt, beispielsweise durch Aufschrauben oder Aufschweißen. Die Befestigungsplatte 22 weist ein in senkrechter Richtung verlaufendes Langloch 24 auf. Auf dem Wagen 23 sind die Geräte 21, 21', 21 " aufeinander gestapelt. Die Geräte 21, 21', 21 " weisen jeweils ein im Wesentlichen allseitig geschlossenes Gehäuse 25, 25', 25" auf. Dabei passt jeweils eine Oberseite des Gehäuses 25', 25" eines Geräts 21', 21" mit einer Unterseite des Gehäuses 25, 25' des jeweiligen darauf gestapelten Geräts 21, 21' formschlüssig zusammen. Zur weiteren Sicherung der Geräte 21, 21', 21 " auf dem Wagen 23 liegen diese mit einer Rückwand 26, 26', 26" an der Befestigungsplatte 22 an und können mit Hilfe jeweils eines Tragbolzens an dieser fixiert werden (s. Fig. 7).

In Fig. 5 ist als weiteres Beispiel für die Anordnung einer als Befestigungsschiene ausgebildeten Befestigungsplatte 22 ein an einer Raumdecke befestigter, schwenkbarer Deckenarm 38 gezeigt, an dessen unterem Ende eine Befestigungsplatte 22 angebracht ist, an der Geräte 25, 25' wie oben beschrieben fixiert werden können. Zur Vereinfachung der Anbringung der Geräte 25, 25' kann am unteren Ende der Befestigungsplatte 22 eine Konsole 39 befestigt sein. Die Befestigungsplatte 2, 22 (s. auch Fig. 3) kann auch horizontal am Deckenarm 38 angeordnet sein, so dass die Geräte 1,1', 25, 25' hängend an der Befestigungsplatte 2, 22 gehalten werden.

In Fig. 6 ist ein Tragbolzen 27, mit dem ein Gerät 21 an der als Befestigungsschiene ausgebildeten Befestigungsplatte 22 fixiert werden kann, dargestellt. Der Tragbolzen 27 weist einen gerätefernen Teilabschnitt 28 und einen gerätenahen Teilabschnitt 28' auf. Der geräteferne und der gerätenahe Teilabschnitt 28, 28' weisen jeweils in einander gegenüber liegenden Teilbereichen ihres Umfangs ein Außengewinde 29, 29' auf und sind in den dazwischen liegenden Teilbereichen ihres Umfangs mit Abflachungen 30, 30' versehen. Bezüglich einer Längsachse des Tragbolzens 27 sind die Abflachungen 30' gegenüber den Abflachungen 30 um 90° versetzt; das Gleiche gilt für den Bereich des Außengewindes 29' gegenüber dem Bereich des Außengewindes 29.

Wie in Fig. 6 schematisch dargestellt ist, kann der Tragbolzen 27 mit seinem gerätenahen Teilabschnitt 28' in ein Langloch 31 der Rückwand 26 des Gehäuses 25, das die Tragstruktur des Geräts 21 bildet, eingesetzt werden. Hier kann der Tragbolzen 27 beispielsweise durch eine auf das Außengewinde 29' vom Geräteinneren her aufgeschraubte Mutter mit dem Gerät 21 verbunden werden; für die Montage an der Befestigungsplatte kann der Tragbolzen 27 dabei innerhalb des Langlochs 31 verschiebbar bleiben (nicht dargestellt). Zur Fixierung des Geräts 21 an der Befestigungsplatte 22 wird der Tragbolzen 27 durch das Langloch 24 der Befestigungsplatte 22 geführt und kann dort durch eine auf das Außengewinde 29 aufgeschraubte Mutter gehalten werden. Die Abflachungen 30, 30' erlauben dabei ein Einsetzen des Tragbolzens 27 in das Langloch 24 ebenso wie in das Langloch 31 und ein Verschieben des Tragbolzens 27 im betreffenden Langloch 24, 31, so dass das Gerät 21 in jeder gewünschten Position platziert und durch Festziehen der auf das Gewinde 29 aufgesetzten Mutter in dieser Position fixiert werden kann. Dabei kann es ausreichend sein, dass auf der Innenseite der Rückwand 26 eine Mutter auf das Gewinde 29' aufgeschraubt ist und auf der vom Gerät 25 abgewandten Seite der Befestigungsplatte 22 eine weitere Mutter auf das Gewinde 29 des Tragbolzens 27 aufgeschraubt ist. Durch Festziehen der letzteren Mutter, die von außen zugänglich ist, kann das Gerät sicher an der Befestigungsplatte fixiert werden. Die geräteferne Stirnseite des Tragbolzens 27 weist eine Mehrzahl von Kontaktbuchsen 32 auf, in die entsprechende Kontaktstifte eines in Fig. 5 nicht dargestellten Steckers einsetzbar sind. Elektrische Leitungen, die mit den Kontaktbuchsen 32 in Verbindung stehen, können innerhalb des Tragbolzens 27 zu entsprechenden Kontakten innerhalb des Geräts 25 geführt sein, oder es können auf der dem Gerät zugewandten Stirnseite des Tragbolzens 27 weitere Kontakte zur Verbindung mit entsprechenden geräteinternen elektrischen Leitungen angeordnet sein (in Fig. 6 nicht gezeigt). Der Tragbolzen 27 kann als ein vom Gerät 25 separates Zwischenstück zur mechanischen Halterung und zum elektrischen Anschluss des Geräts 25 ausgebildet sein.

In Fig. 7 ist beispielhaft eine Anordnung von zwei medizinischen Geräten 25, 25' gezeigt, die an der Befestigungsplatte 22 dadurch fixiert sind, dass auf die gerätefernen Enden 33, 33' der Tragbolzen Muttern 34, 34' aufgeschraubt und über Unterlegscheiben 20, 20' gegen die Rückwand 26, 26', die Teil der Tragstruktur des jeweiligen Geräts 21, 21' ist, festgezogen sind. Auch bei dem in Fig. 7 gezeigten Ausführungsbeispiel ist auf einfache Weise ein separater Austausch der Geräte 21, 21' sowie weiterer Geräte, die an den frei gebliebenen Positionen zwischen den Geräten 21, 21' befestigt und angeschlossen werden können, möglich. Aufgrund der Fixierung in variabler Höhe innerhalb des Langlochs 24 können Geräte unterschiedlicher Höhe einfach und platzsparend fixiert werden. Zusätzlich zu den Gehäusen 25, 25' kann ein Um-Gehäuse vorgesehen sein, um die Geräte 21, 21' zusätzlich gegen Kontamination zu schützen (nicht dargestellt).

In Fig. 8 ist schematisch der geräteferne Teilabschnitt 28 eines Tragbolzens 27 gezeigt, der durch das Langloch 24 einer Befestigungsplatte 22 hindurchgeführt ist. Das medizinische Gerät 21 kann hierdurch, wenn eine in Fig. 8 nicht gezeigte Mutter auf das Außengewinde 29 aufgesetzt und festgezogen ist, an der Befestigungsplatte 22 mechanisch gehalten werden. Die geräteferne Stirnseite 35 weist eine Mehrzahl von Kontaktbuchsen 32 auf, in die entsprechende Kontaktstifte 36 eines Steckers 37 einsetzbar sind. Der Tragbolzen 27 dient somit nicht nur zum mechanischen Halten des Geräts 21, sondern auch zum Verbinden mit einer elektrischen Verbindungsleitung durch Aufstecken des Steckers 37 der Verbindungsleitung auf das geräteferne Ende des Tragbolzens 27.

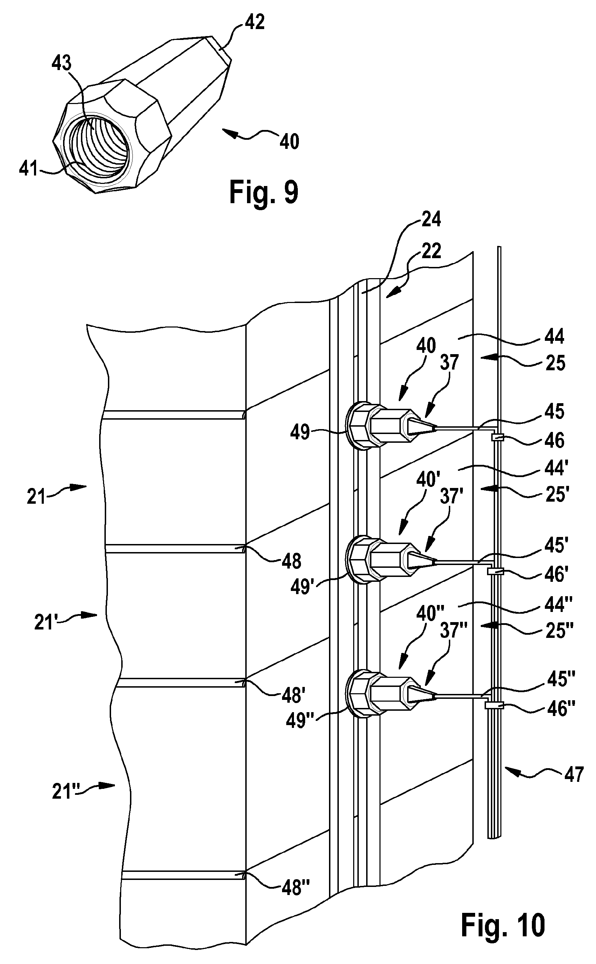

In Fig. 9 ist eine als Überwurfmutter ausgebildete Mutter 40 gezeigt, die sowohl zur mechanischen Fixierung des medizinischen Geräts an der Befestigungsplatte 2, 22 (s. Fig. 1 bis 8) als auch zur Sicherung eines auf den Tragbolzen 7, 27 aufsteckbaren elektrischen Steckers 37 an dem Tragbolzen 7, 27 dient. Die Mutter 40 weist in einem vorderen Bereich ein Innengewinde 41 auf, mit dem sie auf ein Außengewinde 29 eines gerätefernen Teilabschnitts 28 des Tragbolzens 7, 27 aufschraubbar ist (s. Fig. 6 und 8). In ihrem hinteren Bereich weist die Mutter 40 eine Verjüngung 42 auf, durch die die Durchgangsbohrung 43 der Mutter 40 derart verjüngt ist, das ein Stecker einer Verbindungsleitung innerhalb der Durchgangsbohrung 43 gehalten wird. Zum Aufschrauben auf den Tragbolzen 7, 27 ist die Mutter 40 an ihrer Außenseite mit Abflachungen beispielsweise in Form eines Achtkants versehen. Die Mutter 40 kann derart ausgestaltet sein, dass zum Festziehen und Lösen ein Spezialwerkzeug erforderlich ist; hierdurch können ein unautorisiertes Lösen der Mutter 40 und ein unbefugtes Entfernen des Geräts verhindert werden. Andererseits kann die Mutter 40 auch derart ausgebildet sein, dass sie ohne ein Werkzeug festgezogen und wieder gelöst werden kann; hierdurch wird eine besonders einfache Montage des Geräts an der Trägervorrichtung ermöglicht.

Wie in Fig. 10 gezeigt, ist es gemäß einem Ausführungsbeispiel der Erfindung vorgesehen, dass eine Mehrzahl von medizinischen Geräten 21, 21', 21 " aufeinander gestapelt werden können. Dabei steht jeweils von einer Seitenwand 44, 44', 44" des Gehäuses 25, 25', 25" des Geräts 21, 21', 21 " ein Tragbolzen ab, der durch das vertikal verlaufende Langloch 24 einer Befestigungsplatte 22 hindurchgeführt und durch die als Überwurfmutter ausgebildete Mutter 40, 40', 40" an der Befestigungsplatte 22 gesichert werden kann. Zur Verbesserung der Auflage der Mutter 40, 40', 40" an der Befestigungsplatte 22 ist zwischen der Mutter 40, 40', 40" und der Befestigungsplatte 22 jeweils eine Unterlegscheibe 49, 49', 49" angeordnet. Die Mutter 40, 40', 40" ist an ihrem vom jeweiligen Gerät 21, 21', 21" abgewandten Ende derart verjüngt, dass durch Aufschrauben auf den Tragbolzen der Stecker 37, 37', 37" der zum jeweiligen Gerät 21, 21', 21" geführten Verbindungsleitung 45, 45', 45 " auf dem Tragbolzen gehalten wird. Hierdurch wird es ermöglicht, dass ohne weitere Arbeitsschritte durch das mechanische Fixieren des Geräts 21, 21', 21 " an der Befestigungsplatte 22 auch eine elektrische Verbindung hergestellt und gegen Lösen gesichert wird. Die Verbindungsleitungen 45, 45', 45" können etwa durch Kabelbinder 46, 46', 46" zusammengehalten werden und einem Kabelbaum 47 eines OP-Steuerungssystems angehören. Die Verbindungsleitungen 45, 45', 45" umfassen beispielsweise 5 V-, 12 V- oder 24 V-Versorgungsleitungen sowie Bus-, Video- und/oder Audio-Signalleitungen und weitere Datenleitungen zum Betreiben der Geräte 21, 21', 21 " mit Hilfe einer zentralen Steuerung bzw. Recheneinheit.

Vorzugsweise kann jeweils ein Gerät 21, 21' mit seiner Unterseite formschlüssig auf die Oberseite des darunter angeordneten Geräts 21', 21 " gestellt werden. An einer Unterkante einer Vorderseite des Gehäuses des Geräts 21, 21', 21" ist jeweils eine Lippe 48, 48', 48" derart angeordnet, dass die Lippe 48, 48', 48" die Oberkante der Vorderseite des Gehäuses des jeweils darunter stehenden Geräts nach vorne überragt und formbündig dachziegelartig überlappt. Hierdurch kann das Eindringen von Flüssigkeiten, die bei der Benutzung der Geräte, beispielsweise bei einer chirurgischen Operation, an die Vorderseite der Geräte spritzen und zwischen die betreffenden Geräte gelangen könnte, auch ohne ein Um-Gehäuse weitestgehend vermieden werden. Wie in Fig. 10 angedeutet, können mehr als drei Geräte der beschriebenen Art gestapelt werden.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1,1': Gerät
- 2: Befestigungsplatte
- 3: Wagen
- 4: Loch
- 5: Gehäuse
- 6: Rückwand
- 7: Tragbolzen
- 8: Außengewinde
- 9: Mutter
- 10: Unterseite
- 11: Bolzenkörper
- 12: Stirnseite
- 13: Kontaktstift
- 14: Deckenhalter
- 15: Unterlegscheibe
- 20, 20': Unterlegscheibe
- 21, 21', 21 ": Gerät
- 22: Befestigungsplatte
- 23: Wagen
- 24: Langloch
- 25, 25', 25": Gehäuse
- 26, 26', 26": Rückwand
- 27, 27': Tragbolzen
- 28, 28': Teilabschnitt
- 29, 29': Außengewinde
- 30, 30': Abflachung
- 31: Langloch
- 32: Kontaktbuchse
- 33, 33': Gerätefernes Ende
- 34, 34': Mutter
- 35: Stirnseite
- 36: Kontaktstift
- 37: Stecker
- 38: Deckenarm
- 39: Konsole
- 40, 40', 40": Mutter
- 41: Innengewinde
- 42: Verjüngung
- 43: Durchgangsbohrung
- 44, 44', 44": Stecker
- 45, 45', 45 ": Verbindungsleitung
- 46, 46', 46": Kabelbinder
- 47: Kabelbaum
- 48, 48', 48": Lippe
- 49, 49', 49": Unterlegscheibe

## Patentansprüche

1. Medizinisches Gerät zur Verwendung in einer medizinischen Anwendungsumgebung, das Haltemittel zum Halten des Geräts (1, 1', 21, 21', 21") an einer der Anwendungsumgebung zugeordneten senkrechten Trägervorrichtung sowie Anschlussmittel zum Anschließen des Geräts (1, 1', 21, 21', 21") an mindestens eine elektrische Verbindungsleitung (45, 45", 45") umfasst, wobei die Haltemittel als mindestens ein mit einer Tragstruktur des Geräts (1, 1', 21, 21', 21") verbundender Tragbolzen (7, 27, 27') ausgebildet sind, wobei das Gerät ein Gehäuse aufweist und der Tragbolzen von einer Wand des Gehäuses nach außen absteht, wobei der Tragbolzen (7, 27, 27') die Anschlussmittel aufweist, **dadurch gekennzeichnet, dass** der Tragbolzen (7, 27, 27') mit einer Rückwand des Gehäuses einstückig ausgebildet ist oder mit dieser verschweißt ist.

2. Medizinisches Gerät nach Anspruch 1, **dadurch gekennzeichnet, dass** das Gerät (1, 1', 21, 21', 21") nur einen einzigen Tragbolzen (7, 27, 27') aufweist.

3. Medizinisches Gerät nach Anspruch 1 oder 2, **dadurch gekennzeichnet, dass** die Anschlussmittel als an einer vom Gerät (1, 1', 21, 21', 21") entfernten Stirnseite des Tragbolzens (7, 27, 27') angeordnete Steckkontakte ausgebildet sind,

4. Medizinisches Gerät nach einem der vorhergehenden Ansprüche, **dadurch gekennzeichnet, dass** der Tragbolzen (7, 27, 27') zumindest abschnittsweise ein Außengewinde (29, 29') aufweist.

5. Medizinisches Gerät nach einem der vorhergehenden Ansprüche, **dadurch gekennzeichnet, dass** der Tragbolzen (7, 27, 27') in einem gerätefernen Bereich des Tragbolzens (7, 27, 27') zwei bezüglich einer Längsachse des Tragbolzens (7, 27, 27') einander gegenüberliegende zweite Abflachungen (30) aufweist.

6. Medizinisches Gerätesystem, umfassend eine Mehrzahl medizinischer Geräte (1, 1', 21, 21', 21") gemäß einem der vorhergehenden Ansprüche sowie eine Trägervorrichtung zum Halten der Mehrzahl der medizinischen Geräte (1, 1', 21, 21', 21"), wobei die Trägervorrichtung eine mit mindestens einem Durchbruch versehene Befestigungsplatte (2, 22) aufweist und wobei die Tragbolzen (7, 27, 27') der Mehrzahl der medizinischen Geräte (1, 1', 21, 21', 21") zum Befestigen in dem mindestens einen Durchbruch der Befestigungsplatte (2, 22) ausgebildet sind.

7. Medizinisches Gerätesystem nach dem vorhergehenden Anspruch, **dadurch gekennzeichnet, dass** der Durchbruch der Befestigungsplatte (2, 22) als Loch (4) eines Lochrasters oder als Langloch (24) ausgebildet ist.

8. Medizinisches Gerätesystem nach Anspruch 6 oder 7, **dadurch gekennzeichnet, dass** das Gerätesystem eine auf einen durch den Durchbruch hindurchführbaren Bereich des Tragbolzens (7, 27, 27') aufschraubbare Mutter (40, 40', 40") zum Fixieren des mindestens einen medizinischen Geräts (1, 1', 21, 21',21") an der Befestigungsplatte (2, 22) umfasst.

9. Medizinisches Gerätesystem nach einem der Ansprüche 6 bis 8, **dadurch gekennzeichnet, dass** mindestens ein erstes Gerät (21, 21') eine Unterseite und mindestens ein zweites Gerät (21', 21") eine Oberseite aufweist, wobei die Unterseite und die Oberseite derart ausgebildet sind, dass das erste Gerät (21, 21') mit seiner Unterseite Formschlüssig auf die Oberseite des zweiten Geräts (21', 21") aufsetzbar ist.

10. Medizinisches Gerätesystem nach dem vorhergehenden Anspruch, **dadurch gekennzeichnet, dass** das erste und das zweite Gerät (21, 21', 21") jeweils ein im Wesentlichen allseitig geschlossenes Gehäuse (25, 25', 25") mit einer Vorderwand aufweisen und dass mindestens das erste Gerät (21, 21') an einer Unterkante der Vorderwand eine Lippe (48, 48') trägt, die dann, wenn das erste Gerät (21, 21') auf das zweite Gerät (21', 21") aufgesetzt ist, eine Oberkante der Vorderwand des zweiten Geräts (21', 21 ") überlappt.

11. Medizinisches Gerätesystem nach dem vorhergehenden Anspruch, **dadurch gekennzeichnet, dass** das Gehäuse (25, 25', 25") des ersten und des zweiten Geräts (21, 21', 21") jeweils eine Rückwand und/oder eine Seitenwand (44, 44', 44") aufweist, wobei aus der Rück- bzw. Seitenwand der Tragbolzen (27, 27') des jeweiligen Geräts (21, 21', 21") hervorsteht.

## Claims

1. Medical appliance for use in a medical environment, comprising holding means for holding the appliance (1, 1', 21, 21', 21") on a vertical support device assigned to the medical environment, and attachment means for attaching the appliance (1, 1', 21, 21', 21") to at least one electrical connection line (45, 45', 45"), wherein the holding means are designed as at least one support bolt (7, 27, 27') connected to a support structure of the appliance (1, 1', 21, 21', 21"), wherein the appliance has a housing and the support bolt juts out from a wall of the housing, wherein the support bolt (7, 27, 27') has the attachment means, **characterized in that** the support bolt (7,27, 27') is formed integrally with a rear wall of the housing or is welded onto said rear wall.

2. Medical appliance according to Claim 1, **characterized in that** the appliance (1, 1', 21, 21', 21") has only a single support bolt (7, 27, 27').

3. Medical appliance according to Claim 1 or 2, **characterized in that** the attachment means are designed as plug contacts arranged on an end face of the support bolt (7, 27, 27') directed away from the appliance (1, 1', 21, 21', 21").

4. Medical appliance according to one of the preceding claims, **characterized in that** the support bolt (7, 27, 27') has an outer thread (29, 29') at least in some parts.

5. Medical appliance according to one of the preceding claims, **characterized in that** the support bolt (7, 27, 27') has, in an area of the support bolt (7, 27, 27') directed away from the appliance, two second flat surfaces (30) lying opposite each other with respect to a longitudinal axis of the support bolt (7, 27, 27').

6. Medical appliance system comprising a plurality of medical appliances (1, 1', 21, 21', 21") according to one of the preceding claims and a support device for holding the plurality of medical appliances (1, 1', 21, 21', 21"), wherein the support device has a fastening plate (2, 22) provided with at least one aperture, and wherein the support bolts (7, 27, 27') of the plurality of medical appliances (1, 1', 21, 21', 21") are designed for fastening in the at least one aperture of the fastening plate (2, 22).

7. Medical appliance system according to the preceding claim, **characterized in that** the aperture of the fastening plate (2, 22) is designed as a hole (4) in a grid-like pattern of holes or as an oblong hole (24).

8. Medical appliance system according to Claim 6 or 7, **characterized in that** the appliance system comprises a nut (40, 40', 40") which can be screwed onto an area of the support bolt (7, 27, 27') that can be guided through the aperture, which nut (40, 40', 40") is used to fix the at least one medical appliance (1, 1', 21, 21', 21") on the fastening plate (2, 22).

9. Medical appliance system according to one of Claims 6 to 8, **characterized in that** at least a first appliance (21,21') has a bottom face and at least a second appliance (21', 21") has a top face, wherein the bottom face and the top face are designed in such a way that the bottom face of the first appliance (21,21') can be placed with a form fit onto the top face of the second appliance (21', 21 ").

10. Medical appliance system according to the preceding claim, **characterized in that** the first and second appliances (21, 21', 21") each have a substantially fully closed housing (25, 25', 25") with a front wall, and **in that** at least the first appliance (21, 21') carries, on a lower edge of the front wall, a lip (48, 48') which, when the first appliance (21, 21') is placed onto the second appliance (21', 21"), overlaps an upper edge of the front wall of the second appliance (21', 21 ").

11. Medical appliance system according to the preceding claim, **characterized in that** the housing (25, 25', 25") of the first and second appliances (21, 21', 21") in each case has a rear wall and/or a side wall (44, 44', 44"), wherein the support bolt (27, 27') of the respective appliance (21, 21', 21") protrudes from the rear wall or side wall.

## Revendications

1. Appareil médical pour l'utilisation dans un environnement d'application médicale, qui comprend des moyens de fixation pour fixer l'appareil (1, 1', 21, 21', 21") un dispositif de support vertical associé à l'environnement d'application ainsi que des moyens de raccordement pour raccorder l'appareil (1, 1', 21, 21', 21") à au moins une conduite de connexion (45, 45', 45"), les moyens de fixation étant réalisés sous la forme d'au moins un boulon de support (7, 27, 27') connecté à une structure de support de l'appareil (1, 1', 21, 21', 21"), l'appareil présentant un boîtier et le boulon de support faisant saillie vers l'extérieur depuis une paroi du boîtier, le boulon de support (7, 27, 27') présentant les moyens de raccordement, **caractérisé en ce que** le boulon de support (7, 27, 27') est réalisé d'une seule pièce avec une paroi arrière du boîtier ou est soudé à celle-ci.

2. Appareil médical selon la revendication 1, **caractérisé en ce que** l'appareil (1, 1', 21, 21', 21") présente seulement un boulon de support unique (7, 27, 27).

3. Appareil médical selon la revendication 1 ou 2, **caractérisé en ce que** les moyens de raccordement sont réalisés sous la forme de contacts disposés au niveau d'un côté frontal du boulon de support (7, 27, 27') éloignés de l'appareil (1, 1', 21, 21', 21").

4. Appareil médical selon l'une quelconque des revendications précédentes, **caractérisé en ce que** le boulon de support (7, 27, 27') présente au moins en partie un filetage extérieur (29, 29').

5. Appareil médical selon l'une quelconque des revendications précédentes, **caractérisé en ce que** le boulon de support (7, 27, 27') présente, dans une région éloignée de l'appareil du boulon de support (7, 27, 27'), deux deuxièmes méplats (30) opposés l'un à l'autre par rapport à un axe longitudinal du boulon de support (16, 27, 27').

6. Système d'appareil médical comprenant une pluralité d'appareils médicaux (1, 1', 21, 21', 21") selon l'une quelconque des revendications précédentes ainsi qu'un dispositif de support pour supporter la pluralité d'appareils médicaux (1, 1', 21, 21', 21"), le dispositif de support présentant une plaque de fixation (2, 22) pourvue d'au moins un orifice et les boulons de support (7, 27, 27') de la pluralité d'appareils médicaux (1, 1', 21, 21', 21") étant réalisés en vue de la fixation dans l'au moins un orifice de la plaque de fixation (2, 22).

7. Système d'appareil médical selon la revendication précédente, **caractérisé en ce que** l'orifice de la plaque de fixation (2, 22) est réalisé sous forme de trou (4) d'une trame de trous ou sous forme de trou oblong (24).

8. Système d'appareil médical selon la revendication 6 ou 7, **caractérisé en ce que** le système d'appareil présente un écrou (40, 40', 40") pouvant être vissé sur une région du boulon de support (7, 27, 27') pouvant être guidé à travers l'orifice pour la fixation de l'au moins un appareil médical (1, 1', 21, 21', 21") à la plaque de fixation (2, 22).

9. Système d'appareil médical selon l'une quelconque des revendications 6 à 8, **caractérisé en ce qu'**au moins un premier appareil (21, 21') présente un côté inférieur et au moins un deuxième appareil (21', 21") présente un côté supérieur, le côté inférieur et le côté supérieur étant réalisés de telle sorte que le premier appareil (21, 21') puisse être posé avec son côté inférieur par engagement par correspondance de formes sur le côté supérieur du deuxième appareil (21', 21 ").

10. Système d'appareil médical selon la revendication précédente, **caractérisé en ce que** le premier et le deuxième appareil (21, 21', 21") présentent chacun un boîtier (25, 25', 25") fermé essentiellement de tous les côtés avec une paroi avant et **en ce qu'**au moins le premier appareil (21, 21') porte au niveau d'une arête inférieure de la paroi avant une lèvre (48, 48') qui chevauche une arête supérieure de la paroi avant du deuxième appareil (21', 21") lorsque le premier appareil (21, 21') est posé sur le deuxième appareil (21', 21").

11. Système d'appareil médical selon la revendication précédente, **caractérisé en ce que** le boîtier (25, 25', 25") du premier et du deuxième appareil (21, 21', 21") présente à chaque fois une paroi arrière et/ou une paroi latérale (44, 44', 44"), le boulon de support (27, 27') de l'appareil respectif (21, 21', 21") faisant saillie hors de la paroi arrière ou de la paroi latérale.
